# EUROPEAN PATENT APPLICATION

(11) **EP 4 663 215 A1**
(43) Date of publication of application: **17.12.2025**
(21) Application number: 25181302.8
(22) Date of filing: 06.06.2025
(51) Int. Cl.: A61M 1/02, A61M 1/36

(54) **SYSTEMS AND METHODS FOR DETERMINATION OF CAUSE OF LEUKOREDUCTION FLOW RESTRICTION**

(30) Priority: 10.06.2024 US 202463658276 P
(71) Applicant: Fenwal, Inc., Lake Zurich, IL 60047 (US)
(72) Inventor: KUSTERS, Benjamin, Lake Zurich, 60047 (US)
(74) Representative: Maikowski & Ninnemann Patentanwälte Partnerschaft mbB

(57) **Abstract**

Systems, devices, and methods for processing whole blood are disclosed. The system includes a reusable separation device, a disposable fluid circuit, and a controller configured to detect a flow restriction within a leukoreduction filter and to determine the cause of the flow restriction.

## Description

### FIELD OF DISCLOSURE

The present disclosure relates generally to blood processing systems, devices, and methods including a reusable separation device and a disposable fluid circuit. More particularly, the present disclosure relates to a blood processing system configured to detect a flow restriction within a leukoreduction filter and to determine its cause.

### BACKGROUND

Various blood processing systems now make it possible to collect particular blood constituents (e.g., red blood cells and platelets), instead of whole blood, from a blood source. Typically, in such systems, whole blood is drawn from a blood source, the particular blood component or constituent is separated, removed, and collected, and the remaining blood constituents are returned to the blood source. Removing only particular constituents is advantageous when the blood source is a human donor, because potentially less time is needed for the donor's body to return to pre-donation levels, and donations can be made at more frequent intervals than when whole blood is collected.

During certain procedures the separated blood component may be leukoreduced. Leukoreduction is the removal of white blood cells (WBCs) from blood components. Typically, leukoreduction is accomplished using filters which retain WBCs while allowing the passage of the collected blood components, such as red blood cells (RBCs) or platelets (PLTs). It is known, however, that filters may become clogged and restrict the flow of components. Clogging may be caused by fibrin clots, sickle and other deformed cell types, and platelet aggregation. Leukoreduction is typically achieved using gravity forced flow of fluid through a filter. Thus, the formation of a clog or other restriction reduces or even stops the flow of the blood components through the filter as the driving pressure of gravity-forced flow is not strong enough to overcome the flow restriction, leading to insufficient leukoreduction or leukoreduction failure.

U.S. Patent Application Serial No. 17/909,799 describes an automated blood or biological fluid processing system wherein blood components are forced through a filter by pump driven flow and thus, the driving pressure may be sufficient to overcome flow restrictions to a much greater degree than gravity-forced flow. Yet, if the filter pressure becomes too great due to the flow restriction, leukoreduction failure or breakage of the kit components of the disposable fluid flow circuit may occur. Accordingly, in the instance of leukoreduction failure, the collected blood component may contain undesirable WBCs potentially including sickle and other deformed cell types, thus making the collected blood component unusable.

Therefore, there exists a need for improved blood separation systems, devices, and methods for monitoring the pressure within a leukoreduction filter and determining the cause of a flow restriction within a leukoreduction filter and adjusting the fluid processing.

### SUMMARY

There are several aspects of the present subject matter which may be embodied separately or together in the devices, systems, and methods described and/or claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto or later amended. For purposes of this description and claims, unless otherwise expressly indicated, "blood" is intended to include whole blood and blood components, such as concentrated red cells, plasma, platelets and white cells, whether with or without anticoagulant or additives.

In one aspect, a system for collecting separated blood components from whole blood is disclosed. The system may include a disposable fluid flow circuit that includes a separation chamber, a source of biological fluid, a container for receiving a separated blood component, a flow path between said separation chamber, and said container. A leukoreduction filter is located downstream of the separation chamber and upstream of said container. The system also includes a durable hardware component for receiving the disposable fluid circuit. The durable hardware component includes a separator configured to receive the separation chamber and a pressure sensor configured to detect the pressure within the leukoreduction filter. The system includes a controller configured to automatically operate the system to perform one or more blood processing procedures selected by an operator; wherein the controller is further configured to receive information from the pressure sensor, measure a flow restriction within the leukoreduction filter based on the information from the pressure sensor, and determine a cause of the flow restriction.

In another aspect, a method of separating whole blood into its constituent blood components is disclosed. The method includes separating whole blood in a whole blood separation system. The whole blood separation system includes a disposable fluid flow circuit, a separation chamber, a fluid flow control cassette in fluid communication with the separation chamber, a plurality of containers in fluid communication with the fluid flow control cassette and a leukoreduction filter located downstream of the separation chamber. The system further includes a durable hardware component that includes a separator configured to receive the separation chamber and a pressure sensor configured to detect the pressure within the leukoreduction filter. A controller configured to automatically operate the system to perform one or more blood processing procedures selected by an operator is included. The controller is further configured to receive information from the pressure sensor, measure a flow restriction within the leukoreduction filter based on the information from the pressure sensor, and determine a cause of the flow restriction. The method further includes measuring the pressure within the leukoreduction filter and detecting a flow restriction within the leukoreduction filter based on the pressure within the leukoreduction filter. In accordance with the method, flow of the separated blood component to the leukoreduction filter may be restricted or diverted if the pressure within the leukoreduction filter exceeds a predetermined threshold.

These and other aspects of the present subject are set forth in the following detailed description of the accompanying drawings.

### BRIEF DESCRIPTION OF THE DRAWINGS

FIG. 1 is a perspective view of an example of a reusable hardware component of a blood processing system which is configured to receive a disposable fluid flow circuit.
FIG. 2 is a plan view of an example of a disposable fluid flow circuit for use in combination with the durable hardware component of FIG. 1.
FIG. 2A is a plan view of the cassette of the disposable fluid flow circuit showing the internal flow paths and other components of the system.
FIG. 3 is a schematic view of another example of a fluid flow circuit mounted to the processing device of FIG. 1 in a first configuration flowing a separated blood component through a leukoreduction filter.
FIG. 4 is a schematic view of the fluid flow circuit of FIG. 3 in a second configuration flowing a separated blood product to bypass the leukoreduction filter.
FIG. 5 is a flowchart showing a method of determining the cause of a flow restriction within a leukoreduction filter.
FIGS. 6A-C are graphs showing trends/profiles representative of different flow restrictions.

### DETAILED DESCRIPTION OF THE EMBODIMENTS

The embodiments disclosed herein are for the purpose of providing an exemplary description of the present subject matter. They are, however, only exemplary and not exclusive, and the present subject matter may be embodied in various forms. Therefore, specific details disclosed herein are not to be interpreted as limiting the subject matter as defined in the accompanying claims.

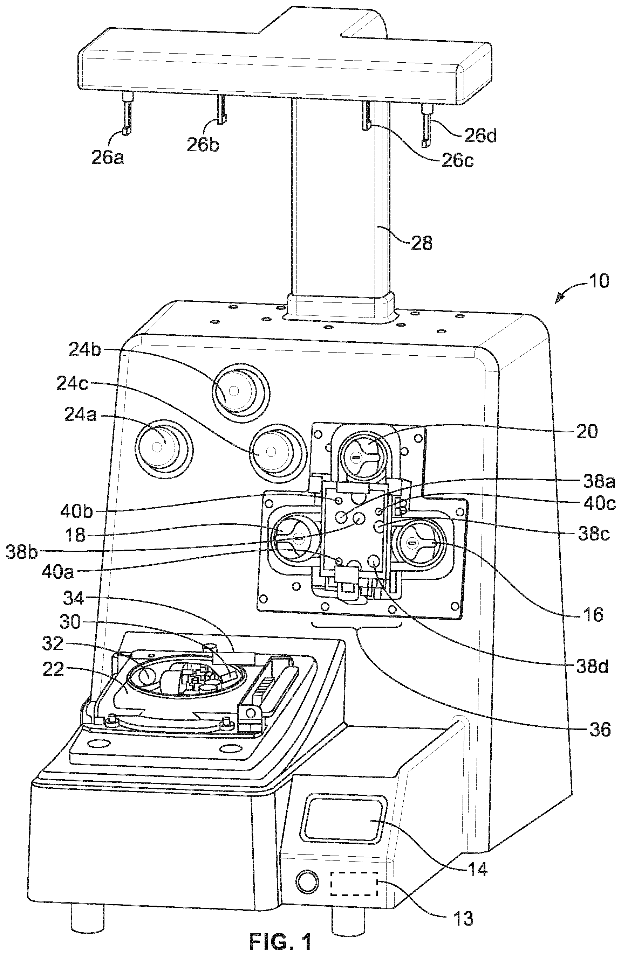

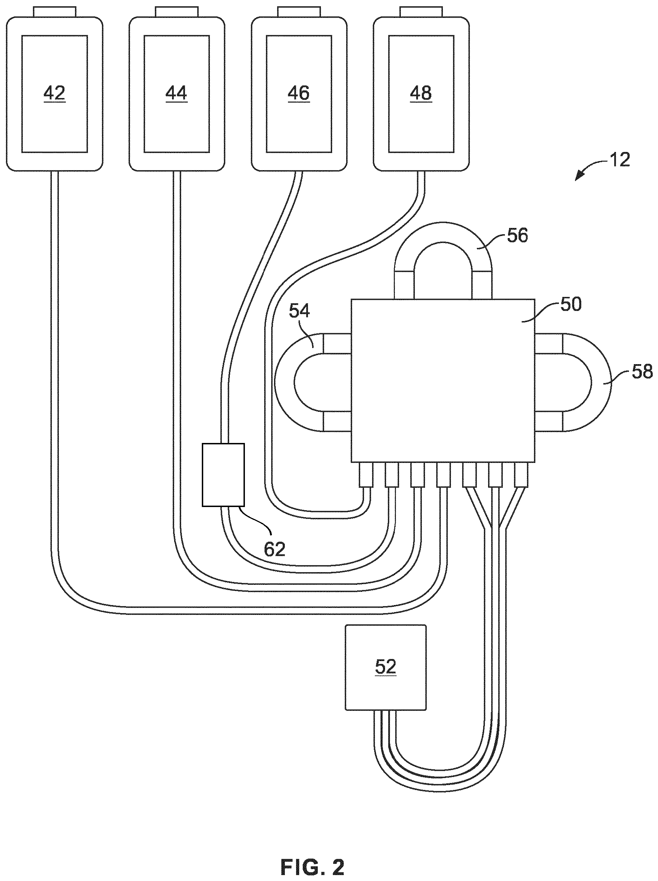

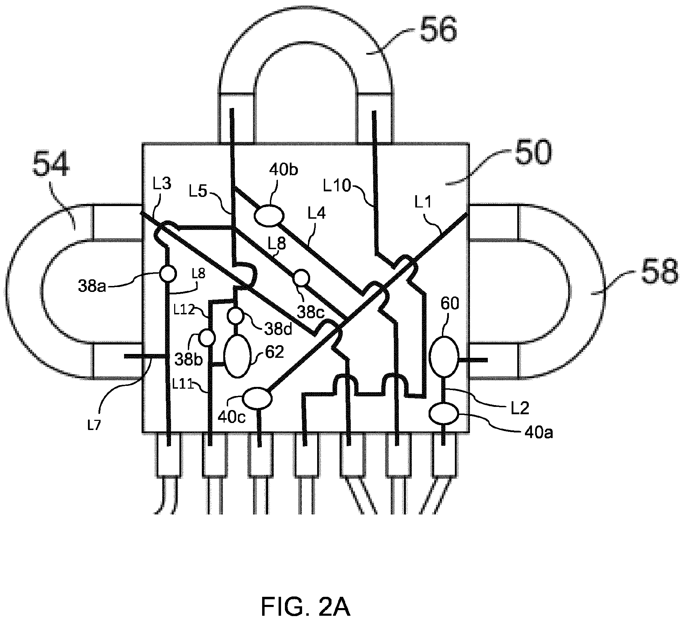

FIG. 1 depicts an example of a reusable, durable hardware component (e.g., processing device) 10 of a blood processing system, while FIG. 2 depicts an example of a disposable or single use fluid flow circuit 12 to be used with the hardware component 10 for processing collected whole blood. In an example, the reusable, durable hardware component 10 can be the device described in International Patent Publication No. WO 2021/194824, which is incorporated herein by reference in its entirety. The illustrated processing device 10 includes associated pumps, valves, sensors, displays and other apparatuses for configuring and controlling flow of fluid through the fluid flow circuit 12, described in greater detail below. The blood processing system may be directed by a controller 13 integral with the processing device 10 that includes a programmable microprocessor to automatically control the operation of the pumps, valves, sensors, etc. The system may also include wireless communication capabilities to enable the transfer of data from the device to the quality management systems of the operator.

More specifically, the illustrated processing device 10 includes a user input and output touchscreen 14, a pump station including a first blood pump 16 (for pumping, e.g., whole blood), a second pump 18 (for pumping, e.g., plasma) and a third pump 20 (for pumping, e.g., additive solution), a separator/centrifuge 22 (which may include or be associated with a centrifuge mounting station and drive unit), and tubing clamps 24a-c. The touchscreen 14 enables user interaction with the processing device 10, as well as the monitoring of procedure parameters, such as flow rates, container weights, pressures, etc. The pumps 16, 18, 20 are illustrated as peristaltic pumps capable of receiving tubing and moving fluid at various rates through the associated conduit dependent upon the procedure being performed. An exemplary centrifuge mounting station/drive unit is disclosed in U.S. Patent No. 8,075,468 (with reference to Figs. 26-28), which is incorporated herein by reference. The clamps 24a-c are capable of opening and closing fluid paths through the tubing or conduits and may incorporate RF sealers to complete a heat seal of the tubing or conduit placed in the clamp to seal the tubing leading to a product container upon completion of a procedure.

The processing device 10 also includes hangers 26a-d (which may each be associated with a weight scale(s)) for suspending the various containers of the disposable fluid flow circuit 12. The hangers 26a-d are preferably mounted to a support 28, which is vertically translatable to improve the transportability of the processing device 10.

An optical system comprising a laser 30 and a photodetector 32 is associated with the centrifuge 22 for determining and controlling the location of an interface between separated blood components within the centrifuge 22. An exemplary optical system is shown in US Patent Application Publication No. 2019/0201916, which is hereby incorporated herein by reference. An optical sensor 34 is also provided to optically monitor one or more conduits leading into or out of the centrifuge 22.

The face of the processing device 10 includes a nesting module 36 for seating a flow control cassette 50 (FIG. 2) of the fluid flow circuit 12 (described in greater detail below). The cassette nesting module 36 is configured to receive various disposable cassette designs so that the system may be used to perform different types of fluid processing procedures. Embedded within the illustrated cassette nesting module 36 are valves 38a-d for opening and closing fluid flow paths within the flow control cassette 50, and pressure sensors 40a-c capable of measuring the pressure at various locations of the fluid flow circuit 12.

With reference to FIG. 2, the illustrated fluid flow circuit 12 includes a plurality of containers 42, 44, 46 and 48, with a flow control cassette 50 and a processing/separation chamber 52 that is configured to be received in the centrifuge 22, all of which are interconnected by conduits or tubing segments, so as to permit continuous flow centrifugation. The flow control cassette 50 routes the fluid flow through three tubing loops 54, 56, 58, with each loop being positioned to engage a particular one of the pumps 16, 18, 20. The conduits or tubing may extend through the cassette 50, or the cassette 50 may have pre-formed fluid flow paths that direct the fluid flow.

In the fluid flow circuit 12 shown in FIG. 2, container 42 may be pre-filled with additive solution, container 44 may be filled with whole blood and connected to the fluid flow circuit 12 at the time of use, container 46 may be an empty container for the receipt of red blood cells (RBCs) separated from the whole blood, and container 48 may be an empty container for the receipt of plasma separated from the whole blood. While FIG. 2 shows a whole blood container 44 (configured as a blood pack unit, for example) as a blood source, it is within the scope of the present disclosure for the blood source to be a living donor.

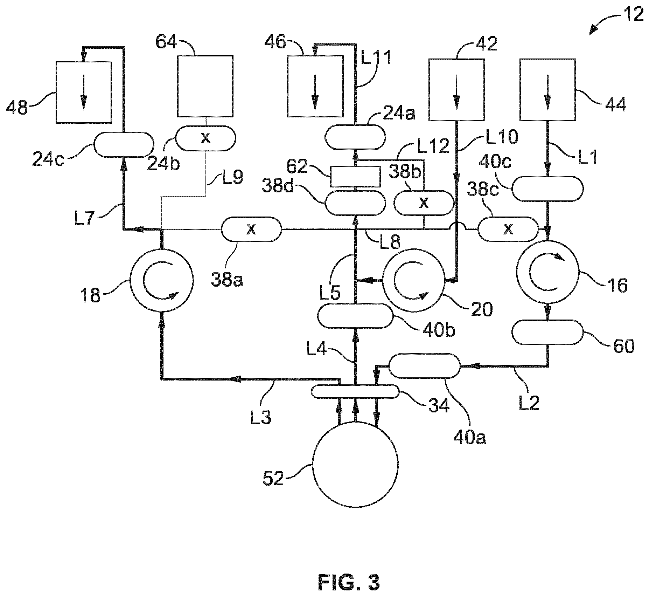

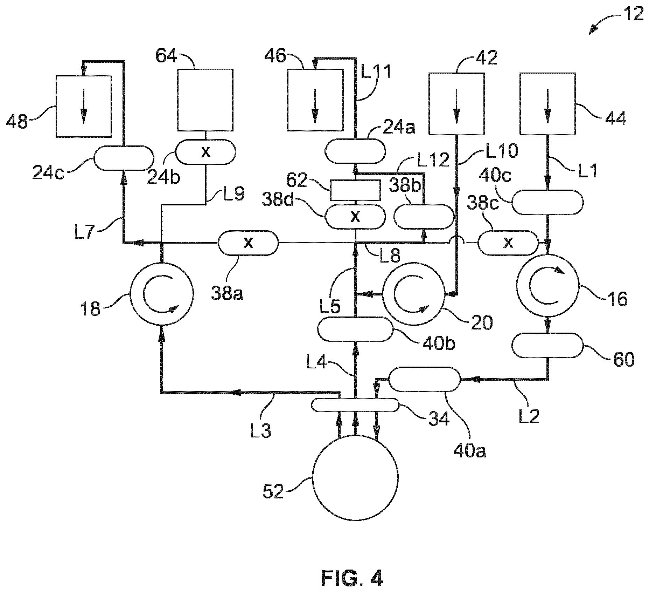

Additionally, the fluid flow circuit 12 includes a leukoreduction filter 62 through which blood such as separated red blood cells are flowed prior to entering the red blood cell collection container 46. The fluid flow circuit may optionally include an air trap 60 (shown in FIGS. 3 and 4) through which the whole blood is flowed prior to entering the separation chamber.

The processing chamber 52 may be pre-formed in a desired shape and configuration by injection molding from a rigid plastic material, as shown and described in US 6,849,039, which is hereby incorporated by reference herein. The specific geometry of the processing chamber 52 may vary depending on the elements to be separated, and the present disclosure is not limited to the use of any specific chamber design. For example, it is within the scope of the present disclosure for the processing chamber 52 to be formed of a generally flexible material, rather than a generally rigid material. When the processing chamber 52 is formed of a generally flexible material, it relies upon the centrifuge 22 to define a shape of the processing chamber 52. An exemplary processing chamber formed of a flexible material and an associated centrifuge are described in U.S. Patent No. 6,899,666, which is hereby incorporated herein by reference.

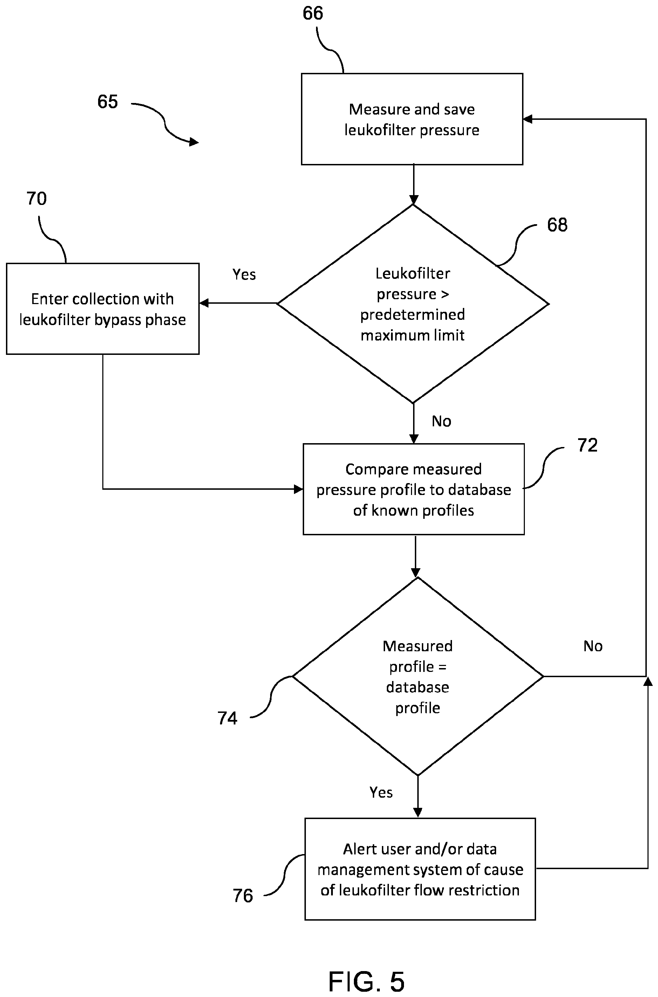

The fluid flow circuit 12 may be variously configured without departing from the scope of the disclosure. The circuit may include additional containers, filters, and/or differently configured tubing/conduits depending on the procedure. For instance, in a red blood cell, plasma, and buffy coat product collection, the disposable circuit includes an additional buffy coat collection container 64 (as shown in FIGS. 4 and 5). Different configurations of the disposable fluid flow circuit 12 and blood processing procedures including, red blood cell and plasma product collection; red blood cell, plasma, and buffy coat product collection; and red blood cell product washing are described in greater detail in International Patent Publication No. WO 2021/194824, which has been incorporated by reference above.

In accordance with the present disclosure, the controller 13 of the processing device 10 may be pre-programmed to automatically operate the system to perform one or more standard blood processing procedures selected by an operator by input to the touchscreen 14 and is configured to be further programmed by the operator to perform additional blood processing procedures. The controller 13 may be pre-programmed to substantially automate a variety of procedures, including, but not limited to: red blood cell and plasma production from a single unit of whole blood, buffy coat pooling, buffy coat separation into a platelet product (as described in US 2018/0078582, which is herein incorporated by reference), glycerol addition to red blood cells, red blood cell washing, platelet washing, and cryoprecipitate pooling and separation.

The pre-programmed blood processing procedures operate the system at pre-set settings for flow rates and centrifugation forces, and the programmable controller 13 may be further configured to receive input from the operator as to one or more of flow rates and centrifugation forces for the standard blood processing procedure to override the pre-programmed settings.

In addition, the programmable controller 13 is configured to receive input from the operator through the touchscreen 14 for operating the system to perform a non-standard blood processing procedure. More particularly, the programmable controller 13 may be configured to receive input for settings for the non-standard blood processing procedure including flow rates and centrifugation forces.

Furthermore, in accordance with the present disclosure, the controller 13 is configured to monitor flow through leukoreduction filter 62 and detect and determine a cause of a flow restriction in the leukoreduction filter 62 associated with the disposable fluid flow circuit 12. For example, the controller 13 may be associated with the pressure sensors 40a-c of the processing device 10. In particular, the controller 13 receives information from a pressure sensor associated with the leukoreduction filter 62 of the disposable fluid flow circuit 12.

As shown in FIGS. 3 and 4, pressure sensor 40b is configured to measure the pressure within the leukoreduction filter 62. As illustrated in FIG. 3, pressure sensor 40b may be located downstream of the centrifuge 22 and upstream of the leukoreduction filter 62. In an example, the pressure sensor 40b measures the pressure within the tubing/conduit leading to the leukoreduction filter 62. The pressure within the tubing/conduit correlates with the pressure within the leukoreduction filter 62.

The controller 13 can take various actions based on information received from the pressure sensor 40b. For example, the controller can be programmed to automatically divert the flow of a separated blood component to bypass the leukoreduction filter 62 for the remainder of a procedure if the pressure detected within the leukoreduction filter 62 exceeds a pre-determined/pre-programmed pressure threshold. By diverting the flow of fluid to bypass the leukoreduction filter 62, damage to or failure of the leukoreduction filter 62 can be prevented.

FIGS. 3 and 4 illustrate an example of fluid flow within the fluid flow circuit 12 before and after the threshold pressure in the leukoreduction filter 62 has been exceeded during a blood processing procedure. In particular, FIGS. 3 and 4 illustrate a fluid flow circuit 12 configured for a red blood cell, plasma, and buffy coat product collection procedure as described in greater detail in International Patent Publication No. WO 2021/194824, previously incorporated by reference herein. Due to the similarity between the fluid flow circuit of FIG. 2 and FIGS. 3 and 4, the components of the fluid flow circuit of FIGS. 3 and 4 corresponding to the above-described components of the fluid flow circuit of FIG. 2 will be identified using the same reference numbers, while new or differently configured components will be identified using new reference numbers. In short, the principal difference between the fluid flow circuit of FIG. 2 and the fluid flow circuit of FIGS. 3 and 4 is that the fluid flow circuit 12 of FIGS. 3 and 4 includes a buffy coat collection container 64 and associated tubing/conduit L9.

With reference to FIG. 3, whole blood is drawn into the fluid flow circuit from the blood source (the whole blood container 44 as illustrated in FIG. 3) via line L1 by operation of the whole blood pump 16. Valve 38c is closed, which directs the blood through pressure sensor 40c and into line L2. The blood passes through air trap 60, pressure sensor 40a, and optical sensor 34 before flowing into the processing chamber 52, which is positioned within the centrifuge 22 of the processing device 10.

During the separation stage, the centrifuge is rotated at a rate that is sufficient to separate blood into packed red blood cells and platelet-poor plasma, with the buffy coat therebetween (e.g., in the range of approximately 4,500 to 5,500 rpm).

Once steady state separation has been established, the controller 13 advances the procedure to a collection stage. The valve system of the processing device is actuated to direct the separated plasma and red blood cells to their respective containers, during which time additional whole blood may be drawn into the fluid flow circuit until a total of one unit of whole blood has been drawn into the fluid flow circuit 12. Clamp 24b may be closed and the buffy coat may remain in the processing chamber 52 during the collection stage.

In particular, during the collection stage, valve 38c is closed, which causes the whole blood pump 16 to draw additional blood into line L1 from the blood source into line L2 from line L1, with the blood passing though air trap 60, pressure sensor 40a, and optical sensor 34 before flowing into the processing chamber 52, where it is separated into plasma, red blood cells, and buffy coat.

The separated plasma exits the processing chamber 52 via the plasma outlet port and associated line L3. With valve 38a closed, pump 18 directs the plasma from line L3 into line L7, through open clamp 24c, and into the plasma collection container 48.

The separated red blood cells exit the processing chamber 52 via the red blood cell outlet port and associated line L4. The additive pump 20 is operated by the controller 13 to draw an additive solution (which may be ADSOL ^{®} or some other red blood cell additive) from the additive solution container 42 via line L10. The red blood cells flowing through line L4 are mixed with the additive solution flowing through line L10 at a junction of the two lines L4 and L10 to form a mixture that continues flowing into and through line L5. With clamp 24a in an open configuration, the mixture is ultimately directed into the red blood cell collection container 46, but is first conveyed through the leukoreduction filter 62.

The leukoreduction filter 62 is associated with the pressure sensor 40b in that the pressure sensor measures the pressure within the leukoreduction filter 62. The pressure within the leukoreduction filter 62 can be measured indirectly by measuring the pressure within line L5 leading to the leukoreduction filter 62, or, alternatively, the pressure sensor 40b may be configured to directly measure the pressure within the leukoreduction filter 62. As red blood cells pass through the pressure sensor 40b, pressure measurements are recorded and saved by the controller 13.

Cassette 50 includes a plurality of internal flow paths (e.g., L1-L5, L7-L8, and L10-L12) as shown schematically in Fig. 2A. Cassette 50 includes a flexible polymeric sheet or membrane over at least one face of cassette 50 that separates the internal flow paths and other components from the valves and sensors of the cassette nesting module 36. As described above, pressure sensor 40b detects the pressure in flow path L5 and, more particularly, leukoreduction filter 62. (As shown in FIG. 2A, lines L4 and L5 are in fluid communication with each other. As the lines are filled with incompressible fluid, such as a fluid including a separated blood component, pressure changes caused by any flow restrictions within the filter 62 in line L5 will also occur in line L4, where pressure sensor 40b can be located.)

Positive pressure may cause the membrane to expand toward the sensor hardware, which can include a force sensing resistor, load cell, strain gauge, or any other suitable means for measuring the change in force exerted on the sensor hardware by the membrane or a change in displacement of the membrane due to changes of pressure. Once the system is at a steady-state, the pressure in line L5 should typically only change due to changes of the head height in the RBC container 46 and changes in the filter pressure. Since a potential hydrostatic pressure change over an entire procedure is typically small (e.g., < 15 mmHg after the RBC container 46 is full), such change may be disregarded and any larger change in pressure in line L5 is likely attributable to changes in flow path through of the leukoreduction filter 62.

When the controller 13 detects that the pressure in the leukoreduction filter 62 measured by pressure sensor 40b exceeds a pre-determined threshold, the controller 13 can automatically place the system in a configuration to divert fluid flow to bypass the leukoreduction filter 62, as shown in FIG. 4. The pre-determined threshold may be pre-programmed into controller 13 or an operator may input the value into the system using the touchscreen 14 prior to starting the procedure. While maintaining valves 38a and 38c in a closed configuration, the controller 13 operates the device to close valve 38d and open valve 38b. Thus, red blood cells (with additive solution, if present) are diverted to flow from line L5 to line L8, through open valve 38b, and then through lines L12 and L11 to the red blood cell container 46.

The collection stage continues until one unit of whole blood has been drawn into the fluid flow circuit from the blood source. In the case of a whole blood container 44 being used as the blood source (as in the illustrated embodiment) the collection stage will end when the whole blood container 44 is empty. In the case of a living donor (or in the event that the whole blood container 44 is provided with more than one unit of blood), the volumetric flow rate of the whole blood pump 16 may be used to determine when one unit of whole blood has been drawn into the fluid flow circuit 12.

Once the collection stage has been completed, the controller 13 can transition the system to a number of post-collection stages including a red blood cell recovery stage, a buffy coat harvest stage, an additive solution flush stage, an air evacuation stage, and various post-processing stages, as described in greater detail in International Patent Publication No. WO 2021/194824, previously incorporated by reference herein.

Additionally, the controller can determine the source of or reason for a flow restriction. For instance, the controller 13 can be configured to determine the cause of a flow restriction in accordance with method 65, as shown in FIG. 5., by comparing pressure sensor data collected during the procedure to known flow restriction profiles. In particular, during a blood processing procedure, such as the red blood cell, plasma, and buffy coat collection procedure described above, the controller 13 measures and saves the leukoreduction filter pressure measured by the pressure sensor 40b (step 66). The controller can compare the measurements with a pre-determined pressure threshold (step 68). In some embodiments, the pre-determined thresholds may be pre-programmed to controller 13 and selected by a user and/or empirically derived from historical data and selected by or entered by the user. If the measured pressure does not exceed the pre-determined threshold, the controller may compare the measured and saved data to a database of known pressure/flow restriction profiles (step 72). If the measured pressure exceeds the pre-determined pressure threshold, the controller 13 may automatically place the system in a configuration to divert fluid flow to bypass the leukoreduction filter 62 (step 70), as described above. After diverting the fluid flow, the controller may then proceed to step 72 to determine the cause of the increased pressure/flow restriction.

The controller 13 can access a database of known flow restriction profiles by being pre-programmed to include a database of known flow restriction profiles or, optionally, by being configured to access an external database (e.g., located on the internet or separate storage device) via a wired or wireless connection. Once a flow restriction has been detected (i.e., the pressure sensor detects an increased pressure within the leukoreduction filter 62), the controller 13 can compare the saved pressure data to the database to determine the cause of the flow restriction. The system can then notify/alert a user of the detected flow restriction and of the cause of the flow restriction (step 76). For example, a notification/alert informing the user of a flow restriction and its cause may be displayed on the touchscreen 14.

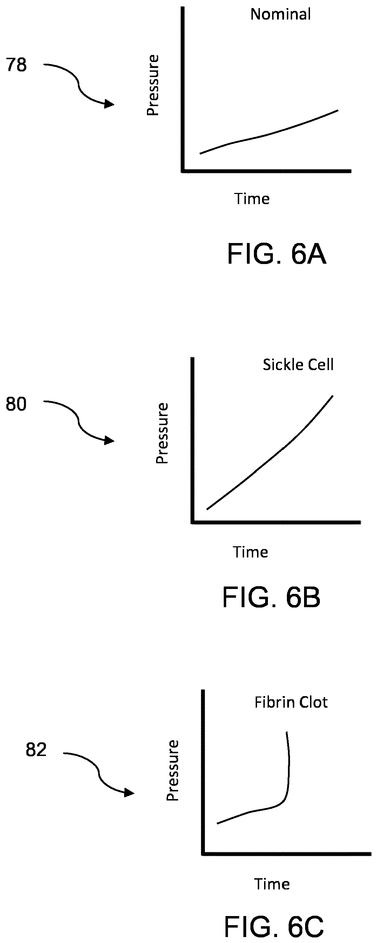

Generally, a leukoreduction filter 62 may be expected to exhibit a known pressure increase throughout the filtration process. For instance, during a procedure the pressure may be expected to increase gradually over time, as illustrated by the graph of FIG. 6A. Nominal pressure data, for example, as shown in FIG. 6A, may be included in the database of known flow restrictions. Accordingly, during a procedure, if the controller 13 determines that the increased leukoreduction filter pressure is caused by the expected nominal pressure increase, controller 13 may continue the procedure without any changes.

When an increase in resistance due to increased flow restriction occurs, the pressure profile will change. Such increased resistance can be caused, for example, by the presence of sickle cells or a fibrin clot. By knowing the cause of or reason for a flow restriction, the user/system operator can determine how to proceed with a particular collected component or product. FIG. 6B illustrates an example of a flow restriction profile caused by the presence of sickle cells. The sickle cell profile exhibits a larger increase (as shown by the greater slope) in pressure over time as the quantity of sickle cells sequestered by the filter increases. If the system determines the filter flow is restricted due to sickle cells or other types of anemias, the user will likely discard the unit.

FIG. 6C illustrates a flow restriction profile caused by a fibrin clot. The fibrin clot profile exhibits an instantaneous and pronounced pressure increase from the nominal profile caused by the clot entering the filter. If the system determines the restricted flow is due to a fibrin clot, the user may attempt a secondary off-line filtration to recover viable RBCs. In another example, if the system detects the restricted flow is due to aggregated platelets the user may want to allow the unit to rest for some time to allow the platelets to un-aggregate before attempting a secondary off-line filtration.

Accordingly, the above-described flow restriction profiles can be included in the flow restriction database. Additionally, various other exemplary pressure profiles (e.g., exponential, cyclic, etc.) representative of known causes of flow restrictions may be included in the flow restriction database without departing from scope of the disclosure.

A blood processing system including hardware component 10 with a controller 13 configured to detect a flow restriction, redirect fluid flow in response to detecting the flow restriction, and determining the cause of the flow restriction as described herein can be used with a variety of fluid flow circuits to perform a variety of blood processing procedures without departing from the scope of the disclosure.

### Other Aspects

There are additional aspects to the methods and systems described herein including, without limitation, the following aspects.

Aspect 1. A system for collecting separated blood components from whole blood including: a disposable fluid flow circuit including a separation chamber, a source of biological fluid, a container for receiving a separated blood component, a flow path between the separation chamber and the container, and a leukoreduction filter located downstream of the separation chamber and upstream of the container; and a durable hardware component for receiving the disposable fluid circuit including a separator configured to receive the separation chamber, a pressure sensor configured to detect the pressure within the leukoreduction filter; and a controller configured to automatically operate the system to perform one or more blood processing procedures selected by an operator; wherein the controller is further configured to receive information from the pressure sensor, measure a flow restriction within the leukoreduction filter based on the information from the pressure sensor, and determine a cause of the flow restriction.

Aspect 2. The system of Aspect 1, wherein the controller is configured to flow the separated blood component through the disposable fluid circuit such that the separated blood component bypasses the leukoreduction filter when the controller measures that the flow restriction exceeds a predetermined threshold.

Aspect 3. The system of any one of Aspects 1-2, wherein the controller is pre-programmed to include pressure information including a nominal filtration profile and profiles representative of other flow restriction causes.

Aspect 4. The system of any one of Aspects 1-3, wherein the controller includes a touchscreen, wherein the touchscreen is configured to receive input from an operator and display procedure information.

Aspect 5. The system of any one of Aspects 1-4, wherein the pressure sensor is located upstream of the leukoreduction filter.

Aspect 6. The system of any one of Aspects 1-4, wherein the leukoreduction filter includes a pressure sensor configured to detect the pressure of a separated blood component entering the leukoreduction filter.

Aspect 7. The system of any one of Aspects 1-6, wherein the blood processing procedure includes red blood cell separation, plasma and buffy coat collection from a single unit of whole blood, buffy coat pooling, buffy coat separation into a platelet product, glycerol addition to red blood cells, red blood cell washing, platelet washing, and cryoprecipitate pooling and separation.

Aspect 8. The system of any one of Aspects 3 through 7 wherein said controller is pre-programmed to include profiles representative of other flow restriction causes including a sickle cell flow restriction profile and a fibrin clot flow restriction profile.

Aspect 9. The system of any one of Aspects 1 through 8 wherein the disposable fluid flow circuit further includes a control cassette in fluid communication with the separation chamber.

Aspect 10. The system of any one of Aspects 1 through 9 wherein said durable hardware component includes one or more pumps for effecting flow through the fluid circuit.

Aspect 11. A method of separating whole blood into its constituent blood components including: separating whole blood in a whole blood separation system configured to execute a blood processing procedure and that includes a leukoreduction filter; measuring the pressure within the leukoreduction filter with a pressure sensor; detecting a flow restriction within the leukoreduction filter based on the pressure within the leukoreduction filter; and restricting or diverting flow of the separated blood component to the leukoreduction filter if the pressure within the leukoreduction filter exceeds a predetermined threshold.

Aspect 12. The method of Aspect 11, further including determining the cause of a flow restriction.

Aspect 13. The method of any one of Aspects 11-12, further including saving the measured pressure within the leukoreduction filter.

Aspect 14. The method of any one of Aspects 11-13, including accessing a database of flow restriction profiles.

Aspect 15. The method of any one of Aspects 12-14, further including comparing the information from the pressure sensor with the database of flow restriction profiles to determine the cause of the flow restriction.

Aspect 16. The method of Aspect 14 wherein the database includes a sickle cell flow restriction profile and a fibrin clot flow restriction profile.

Aspect 17. The method of any one of Aspects 12-16, including alerting a user of the cause of the flow restriction.

Aspect 18. The method of any one of Aspects 11-16, wherein the blood processing procedure includes red blood cell separation, plasma and buffy coat collection from a single unit of whole blood, buffy coat pooling, buffy coat separation into a platelet product, glycerol addition to red blood cells, red blood cell washing, platelet washing, and cryoprecipitate pooling and separation.

Aspect 19. The method of any one of Aspects 15-17, wherein the database includes a nominal flow restriction profile.

Aspect 20. The method of Aspect 19, including continuing the blood processing procedure after detecting that the flow restriction is a nominal flow restriction.

It will be understood that the embodiments and examples described above are illustrative of some of the applications or principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that the claims may be directed to the features thereof, including as combinations of features that are individually disclosed or claimed herein.

## Claims

1. A system for collecting separated blood components from whole blood comprising:
a disposable fluid flow circuit comprising:
a separation chamber;
a source of biological fluid;
a container for receiving a separated blood component;
a flow path between said separation chamber and said container; and
a leukoreduction filter located downstream of the separation chamber and upstream of said container; and
a durable hardware component for receiving the disposable fluid circuit comprising:
a separator configured to receive the separation chamber;
a pressure sensor configured to detect the pressure within the leukoreduction filter; and
a controller configured to automatically operate the system to perform one or more blood processing procedures selected by an operator; wherein the controller is further configured to receive information from the pressure sensor, measure a flow restriction within the leukoreduction filter based on the information from the pressure sensor, and determine a cause of the flow restriction.

2. The system of Claim 1, wherein the controller is configured to flow the separated blood component through the disposable fluid circuit such that the separated blood component bypasses the leukoreduction filter when the controller measures that the flow restriction exceeds a predetermined threshold.

3. The system of any one of Claims 1-2, wherein the controller is pre-programmed to include pressure information including a nominal filtration profile and profiles representative of other flow restriction causes.

4. The system of any one of Claims 1-3, wherein the controller comprises a touchscreen, wherein the touchscreen is configured to receive input from an operator and display procedure information.

5. The system of any one of Claims 1-4, wherein the pressure sensor is located upstream of the leukoreduction filter.

6. The system of any one of Claims 1-4, wherein the leukoreduction filter includes a pressure sensor configured to detect the pressure of a separated blood component entering the leukoreduction filter.

7. The system of any one of Claims 1-6, wherein the blood processing procedure includes red blood cell separation, plasma and buffy coat collection from a single unit of whole blood, buffy coat pooling, buffy coat separation into a platelet product, glycerol addition to red blood cells, red blood cell washing, platelet washing, and cryoprecipitate pooling and separation.

8. The system of any one of Claims 1 through 7 wherein said disposable fluid flow circuit further comprises a control cassette in fluid communication with the separation chamber.

9. The system of any one of Claims 1 through 8 wherein said durable hardware component comprises one or more pumps for effecting flow through said fluid circuit.

10. A method of separating whole blood into its constituent blood components comprising:
separating whole blood in a whole blood separation system configured to execute a blood processing procedure and that includes a leukoreduction filter;
measuring the pressure within the leukoreduction filter with a pressure sensor;
detecting a flow restriction within the leukoreduction filter based on the pressure within the leukoreduction filter; and
restricting or diverting flow of the separated blood component to the leukoreduction filter if the pressure within the leukoreduction filter exceeds a predetermined threshold.

11. The method of Claim 10, further comprising determining the cause of a flow restriction.

12. The method of any one of Claims 10-11, further comprising saving the measured pressure within the leukoreduction filter.

13. The method of any one of Claims 10-12, comprising accessing a database of flow restriction profiles.

14. The method of any one of Claims 11-13, further comprising comparing the information from the pressure sensor with the database of flow restriction profiles to determine the cause of the flow restriction,

15. The method of any one of Claims 11-14, comprising alerting a user of the cause of the flow restriction.

16. The method of any one of Claims 10-14, wherein the blood processing procedure includes red blood cell separation, plasma and buffy coat collection from a single unit of whole blood, buffy coat pooling, buffy coat separation into a platelet product, glycerol addition to red blood cells, red blood cell washing, platelet washing, and cryoprecipitate pooling and separation.
